(19) Europäisches Patentamt
European Patent Office
Office européen des brevets

(11) Publication number: 0 017 507 B1

(12) **EUROPEAN PATENT SPECIFICATION**

(45) Date of publication of patent specification: **04.04.84**

(51) Int. Cl.³: C 07 G 7/00, A 61 K 37/02 //A61K39/395, A61K35/74

(21) Application number: **80301111.3**

(22) Date of filing: **08.04.80**

# (54) Antitumor protein hybrid and process for the preparation thereof.

(30) Priority: **09.04.79 JP 41919/79**

(43) Date of publication of application:
**15.10.80 Bulletin 80/21**

(45) Publication of the grant of the patent:
**04.04.84 Bulletin 84/14**

(84) Designated Contracting States:
**AT BE CH DE FR GB IT LI NL SE**

(56) References cited:
**EP - A - 0 000 938**
**DE - A - 2 456 224**
**US - A - 4 093 607**

**NATURE, vol. 271, 23rd February 1978, pages 752-755 Basingstoke, G.B. P.E. THORPE et al.: "Toxicity of diphtheria toxin for lymphoblastoid cells is increased by conjugation to antilymphocytic globulin"**

(73) Proprietor: **TEIJIN LIMITED**
**11, 1-Chome, Minamihonmachi Higashi-ku**
**Osaka-shi Osaka (JP)**

(72) Inventor: **Masuho, Yasuhiko**
**15-6, Tamdaira 5-chome**
**Hino-shi Tokyo (JP)**
Inventor: **Hara, Takeshi**
**1296-98, Katakura-cho**
**Hachioji-shi Tokyo (JP)**
Inventor: **Noguchi, Teruhisa**
**8-11, Kugenumakaigan 2-chome**
**Fujisawa-shi Kanagawa-ken (JP)**

(74) Representative: **Votier, Sidney David et al,**
**CARPMAELS & RANSFORD 43, Bloomsbury Square**
**London WC1A 2RA (GB)**

(56) References cited:
**MICROBIOLOGY ABSTRACTS, SECTION B, BACTERIOLOGY, vol. 8, no. 5, May 1973, page 164, no. 8B4386 London, G.B. F.L. MOOLTEN et al.: "Antitumor effects of antibody-diphtheria toxin conjugates: use of haptencoated tumor cells as an antigenic target"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 017 507 B1

CHEMICAL ABSTRACTS, vol. 86, no. 17, 25th April 1977, page 220, no. 117226v Columbus, Ohio, U.S.A. TA-MIN CHANG et al.: "Artificial hybrid protein containing a toxic-protein fragment and a cell membrane receptor-binding moiety in a disulfide conjugate. I. Synthesis of diphtheria toxin fragment A-S-S-human placental lactogen with methyl-5-bromovalerimidate"

CHEMICAL ABSTRACTS, vol. 92, no. 23, 9th June 1980, page 670, no. 198268j Columbus, Ohio, U.S.A.

CHEMICAL ABSTRACTS, vol. 89, no. 23, 4th December 1978, no. 192149n Columbus, Ohio, U.S.A. M. YAMAIZUMI et al.: "One molecule of diphtheria toxin fragment A introduced into a cell can kill the cell"

# Antitumor protein hybrid and process for the preparation thereof

## Field of the invention

The present invention relates to a novel antitumor protein hybrid and a process for the preparation thereof. More particularly, the present invention relates to a novel protein hybrid, which, having a moiety which is a fragment having an antibody activity arising from the Fab part of an antitumor immunoglobulin and a moiety which is a fragment of a diphtheria toxin which satisfies the characteristics of fragment A of a diphtheria toxin, is especially useful as a remedy for malignant tumors and to a process for the preparation of the same.

## Description of the prior art

As for the remedies for malignant tumors or cancer (antitumor agents), many drugs have hitherto been known; however, these drugs have the demerit that they cannot be administered enough to destroy tumor cells, because they have a toxic effect not only upon tumor cells but also upon normal cells to a considerable degree. Several attempts have been made to overcome this demerit by combining an antitumor agent or a protein toxin having cytotoxicity with a specific carrier in order to have them selectively absorbed by tumor cells. There exists an antitumor antibody (antitumor immunoglobulin), though very small in amount, in the blood of a cancer patient or on the surface of tumor cells. An antitumor antibody can also be obtained by immunizing an animal with the tumor cells and absorbing the obtained antiserum with the human normal cells. Antitumor antibodies, whether autochthonus, allogeneic, or xenogeneic, are not always capable of displaying a cytotoxic effect against tumor cells; however, they have a common nature of combining with tumor cells with an extremely high selectivity. Antitumor antibodies, therefore, have been used as a carrier to have an antitumor agent or a protein toxin absorbed by tumor cells selectively.

For instance, U.S. Patent 4,093,606 (corresponding to G.B. 1,523,980 and F.R. 2,312,259) discloses, as an antitumor drug, a conjugate of antibody and antitumor drug in which such antitumor drug as daunomycin, etc. is bound covalently with Fab′ dimer of antitumor immunoglobulin. This is superior in that it carries the antitumor drug selectively to the target tumor cells; however, since antitumor drug such as daunomycin, etc. bound with the antibody (Fab′ dimer) exerts cytotoxic effects not only against tumor cells but also against normal cells, it is not satisfactory in view of destroying tumor cells only, and its cytotoxicity itself is not always sufficient either.

Studies have also been made to use diphtheria toxin, which is one of the protein toxins having much stronger toxicity, in the place of an antitumor drug.

For instance, F. L. Moolten et al. report that they prepared a conjugate by conjugating rabbit's anti-SV40-transformed hamster sarcoma or lymphoma antibody to diphtheria toxin with glutaraldehyde as coupling agent and were able to protect hamsters challenged with SV40-transformed tumors by administering the conjugate to hamsters (Journal of the National Cancer Institute, vol. 55, pp 473—477, 1975).

P. E. Thorpe et al. report that the conjugate prepared by coupling diphtheria toxin to antilymphocytic antibody by means of chlorambucil greatly reduced the protein synthesis of human lymphoblastoid cells, CLA4 (Nature, vol. 271, pp 752—754, 1978; see also European Patent Publication No. 0 000 938). The results of these studies show that a conjugate of diphtheria toxin and antibody displays toxicity against the tumor cells selectively. However, these conjugates, when used as an antitumor drug, are supposed to have some demerits as cited below. First, xenogeneic antibody (immunoglobulin) has a strong antigenicity in the human body and induces the formation of anti-xenogeneic immunoglobulic antibody which deactivates the antitumor activity and further causes an anaphylaxis shock. The second of the demerits is that the non-specific toxicity of diphtheria toxin is not nullified. More particularly, the object of these methods is to conjugate diphtheria toxin on the surface of tumor cells by the aid of antitumor antibody; however, since the conjugate contains the whole molecule of diphtheria toxin in its composition, it is apt to bind with normal cell surface receptors for diphtheria toxin and display cytotoxicity against the normal cells. Thirdly comes the defect which is found in the method of cross-linking the antibody with the diphtheria toxin. Some of the crosslinking agents, such as glutaraldehyde or toluene diisocyanate, effect cross-linking not only between the antibody and the toxin but also between antibody and antibody, and toxin and toxin, and moreover, they effect the formation of intra-molecular bonds in the antibody and in the toxin molecule, thus causing the formation of undesirable products and decrease or loss of the antitumor activity.

## Summary of the invention

The present inventors have achieved this invention as a result of their earnest research work to overcome such demerits as found in the prior art by developing an antitumor substance which displays strong cytotoxicity against tumor cells selectively.

The present invention relates to an antitumor protein hybrid which is expressed by the following formula (I):

$$Fab \leftarrow S_1 — (X)_n — S_2 — FA)_m \qquad (I)$$

where

Fab indicates a moiety which is a fragment having an antibody activity arising from the Fab part of an antitumor immunoglobulin;

FA indicates a moiety which is a fragment of a diphtheria toxin which satisfies the characteristics of fragment A of a diphtheria toxin;

X indicates a divalent organic radical derived from N,N'-(1,3-phenylene)-dimaleimide, N,N'-(1,2-phenylene)dimaleimide, N,N'-(1,4-phenylene)dimaleimide, 4,4'-bis(maleoylamino)azobenzene, bis(N-maleimidomethyl)ether, N,N'-alkylenebis(bromoacetamide) or N,N'-alkylenebis-(iodoacetamide) (wherein the alkylene radical has 2—15 carbon atoms), 1,3-dibromoacetone, 1,3-diiodoacetone, N-($\alpha$-bromoacetoxymethyl)maleimide or N-($\alpha$-iodoacetoxymethyl)maleimide;

$S_1$ and $S_2$ are both sulfur atoms, $S_1$ indicating a sulfur atom arising from the disulfide bond (—S—S-bond) in an immunoglobulin and $S_2$ a sulfur atom arising from the disulfide bond in a diphtheria toxin;

n stands for 0 or 1; and

m stands for 1 or 2.

The present invention also relates to a process for preparing said antitumor protein hybrid, which process comprises binding the sulfur atom in said fragment Fab with the sulfur atom in said fragment A directly or indirectly.

Brief description of the drawings

In Fig. 1, (a) is a representation of the basic structure of an immunoglobulin and (b) is a representation of the structure of IgGI of human immunoglobulin.

Fig. 2 is a representation of a diphtheria toxin, wherein (a) shows the structure of intact toxin, (b) shows the structure of nicked toxin, and (c) shows the structures of fragment A and fragment B.

Fig. 3 shows the elution pattern

(—●—)

of a reaction product of fragment Fab' of anti L1210 immunoglobulin IgG and fragment A of diphtheria toxin by column chromatography on Sephadex G 150 superfine and a relationship of the elution pattern vs. ADP ribose transferase activity of EF-2

(——o——),

and concentration of fragment Fab'

(—●—△—o—).

Fig. 4 is an explanatory drawing to show the relationship between the rate of movement in electrophoresis using sodium dodecyl sulfate.

Fig. 5 is a figure to show the elution pattern by Sephadex G150 superfine column chromatography of the reaction product of fragment Fab' having the PDM residue of anti L1210 immunoglobulin IgG and fragment A of diphtheria toxin.

Fig. 6 is a figure to show the result of the analysis of the protein contained at the peaks 1, 2, and 3 indicated in the elution pattern of Fig. 5 conducted according to the Ouchterlony method using guinea pig anti-rabbit Fab' antibody and horse anti-diphtheria toxin antibody.

Fig. 7 is a figure to show the result of the measurement of the cytotoxicity of the antitumor protein hybrid against L1210 with the passage of time.

Description of the preferred embodiments

What is called antitumor immunoglobulin in the present invention is a protein (immunoglobulin) which has an antibody activity (nature to recognize tumor antigen and couple thereto) prepared from, for instance, the serum of a patient with cancer or serum obtained from such animals as monkeys, horses, cows, goats, sheep, rabbits, etc. which are hyperimmunized with cancer cells or cancer antigen according to a publicly known method such as the Cohn ethanol fractionation method, ammonium sulfate fractionation method, ion-exchange chromatography, etc. Or it is a protein having an antibody activity of high selectivity to cancer antigen obtained from a culture fluid of hybridomas or from a serum or ascites of animals inoculated with hybridomas which are prepared by allowing antibody producing lymphocyte obtained by immunizing animals with cancer cells or cancer antigen to fuse, for instance, with myeloma (See, for instance, H. Koprowski, et al., Proc. Natl. Acad. Sci. U.S.A., Vol. 75, No. 7. pp 3405—3409, 1978; K. E. Hellström, et al., Proc. Natl. Acad. Sci. U.S.A., Vol. 76, No. 6, pp 2927—2931, 1979; R. H. Kennett, et al., Science, Vol. 203, pp 1120—1121, 1979.) A protein, which has antibody activity, prepared by isolating an antitumor antibody from a tumor tissue with a denaturant such as surface active agent, etc., followed by the same processing procedure as mentioned above, is also included under the antitumor immunoglobulin according to the present invention.

It is known that there are five major classes of immunoglobulins, IgG, IgA, IgM, IgD and IgE, and

that their basic structure comprises, as shown in Fig. 1, (a), two L chains which are indicated by L in the figure and two H chains indicated likewise by H, all chains being found with at least three disulfide bonds (—S—S— bonds). To explain the basic structure of the immunoglobulin shown in Fig. 1, (a), it consists of two Fab parts which are shown by Fab in the figure and an Fc part shown by Fc; the Fab part has an antibody activity, or more particularly an ability to selectively couple to the antigen; the Fc part has an ability to couple to complements or Fc receptors on the cell membrane.

The moiety Fab which is one of the moieties of the antitumor protein hybrid of the present invention as expressed by formula (I), is a fragment having an antibody activity arising from said Fab part of the immunoglobulin, which fragment is also referred to hereinafter as a "substantial fragment Fab". For instance, it is known that IgGI, which is typical of human immunoglobulins, has a structure shown by Fig. 1, (b) and, when subjected to the papain digestion in the presence of cystine, this immunoglobulin is cleaved on the broken lines A—A′ into two Fab fragments and one Fc fragment as shown in Fig. 1, (b), and the Fab fragments thus obtained can be used as fragment Fab in the present invention. When said IgGI is treated with pepsin, it is cleaved on the broken line B—B′ as shown in Fig. 1, (b), to give a dimer, $(F(ab')_2)$, of the Fab′ part consisting of the Fab part and the hinge part which is shaded with oblique lines in the figure. Two Fab′ fragments can also be obtained by cleaving the disulfide bond in the hinge part reductively, for instance, with the use of a thiol reagent or by cleaving it by means of sulfonation with sulfite ions. Since this Fab′ fragment has an antibody activity like the Fab fragment (though it has no ability, to couple to complements), it can be used as the fragment Fab of the present invention. In the present invention, so far as the fragment Fab has an antibody activity, said Fab fragment or Fab′ fragment may be the one chemically modified.

Thus obtained fragment Fab is used for the preparation of antitumor protein hybrid according to the present invention just as it is, insofar as it has at least one thiol radical (—SH) and/or S-sulfo radical $(—S—SO_3^-)$ in the fragment, but in other cases it is used after it has been converted into a fragment having at least one thiol radical and/or S-sulfo radical by cleaving at least one of the disulfide bonds in the chains (in the H chains or the L chains) and the disulfide bonds between the chains (between the H chains and the L chains) according to publicly known methods. The number of the thiol radicals and/or S-sulfo radicals in the fragment Fab which are formed by cleaving the bonds between the chains is preferably within the range of 1—2 (corresponding to m=1—2 in the formula (I)).

What is called diphtheria toxin in the present invention is a protein toxin produced by Corynebacterium diphtheriae or its mutants. For instance, a diphtheria toxin produced by Corynebacterium diphtheriae consists of a single polypeptide chain having molecular weight of about 62,000—63,000 and this is called an intact toxin. The intact toxin has two disulfide bonds (—S—S— bonds) in its molecule as shown in Fig. 2, (a). When this intact toxin is treated under moderate conditions with such a proteolytic enzyme as trypsin, there occurs a separation at a specific point in the disulfide bond nearer to the amino-terminal to form a nicked toxin as shown in Fig. 2, (b). When this nicked toxin is treated with a reducing agent, it is divided into fragment A having molecular weight of about 24,000 and fragment B having molecular weight of about 38,000—39,000 as shown in Fig. 2, (c). The intact toxin has a very strong toxicity against animals; however, fragment A and fragment B themselves are both nontoxic. On the other hand, the intact toxin has no adnosine diphosphate (ADP)- ribose transferase activity on the elongation factor 2 (EF-2) defined below, while fragment A has the transferase activity. And though fragment B has no transferase activity, it has the capability of coupling to a cell receptor which fragment A does not possess.

In the present invention, the moiety FA, which is one of the moieties of the antitumor protein hybrid as expressed by formula (I), is a fragment of the diphtheria toxin (which fragment is also referred to hereinafter as a "substantial fragment A") that satisfies the aforementioned characteristics of fragment A, namely the following two characteristics:

(1) To have ADP-ribose transferase activity on EF-2.
(2) To have no capability of coupling to a cell receptor and no cytotoxicity by itself.

So far as the abovementioned two requirements are satisfied, nontoxic protein produced by a mutant (which has one disulfide bond in the molecule) such as CRM 30 and CRM 45 produced, for instance, from Corynebacterium diphtheriae mutant such as $C_7$ ($\beta$30) and $C_7$ ($\beta$47) and the fragment obtained by treating them under moderate conditions with trypsin in the presence of such a reducing agent as thiol reagent are included in fragment A of the present invention.

Fragment A thus obtained is used for the preparation of antitumor protein hybrids of the present invention just as it is without being subjected to any treatment when it has at least one thiol radical in the fragment or after its thiol radical is turned into an S-sulfo radical on treatment with sulfite ions. When it has one disulfide bond in the fragment, it is used after conversion into a fragment having at least one thiol radical and/or S-sulfo radical by cleaving the disulfide bond according to a publicly known method.

As for fragment A in the present invention, a fragment having one thiol radical or S-sulfo radical in the molecule is especially preferable.

The ADP-ribose transferase activity on EF-2 is defined as follows.

EF-2 is known as a protein elongation factor which is related with the protein synthesis of cells and fragment A of the diphtheria toxin deactivates EF-2 by catalytically promoting the reaction mentioned below between EF-2 and nicotinamideadeninedinucleotide (NAD):

$$EF\text{-}2+NAD^{+}\rightarrow(ADP\ ribose)-(EF\text{-}2)+\ nicotinamide+H^{+}$$

The performance to promote this reaction is defined as ADP-ribose transferase activity on EF-2.

ADP-ribose transferase activity on EF-2 acts to interfere with protein synthesis and works as an entity of the lethaltoxicity for animals; however, it is necessary for fragment A of the diphtheria toxin which has this ADP-ribose transferase activity on EF-2 to have fragment B which is capable of coupling to a cell receptor in order that fragment A enters into the cell and exerts its cytocidal effect: fragment A alone cannot bring about death in animals.

In the present invention, when fragment Fab of antitumor immunoglobulin having at least one thiol radical and/or S-sulfo radical in the fragment is made to react directly with fragment A of the diphtheria toxin having at least one thiol radical and/or S-sulfo radical in the fragment under the reaction conditions mentioned later, antitumor protein hybrids expressed by the undermentioned formula (II) which corresponds to the aforementioned formula (I) wherein n=0 is obtained.

$$Fab\text{-}(S_1\text{—}S_2\text{—}FA)_m \qquad (II)$$

(where the definitions of Fab, FA, m, $S_1$ and $S_2$ are the same as those given in the case of formula (I)). These hybrids are preferable from the viewpoint of ease of preparation, separation and purification.

In the present invention, the divalent organic radical, which is expressed by X in the case where n=1 in the aforementioned formula (I), means an organic radical arising from a cross-linking agent having in the molecule at least two functional groups capable of forming a sulfide bond (—S— bond) by reaction with a thiol radical (—SH). These cross-linking agents are the bismaleimide compounds N,N′-(1,3-phenylene)-dimaleimide, N,N′-(1,2-phenylene)dimaleimide, N,N′-(1,4-phenylene)dimaleimide, 4,4′-bis(maleoylamino)azobenzene and bis(N-maleimidomethyl)ether; the bishalocarbonyl compounds N,N′-alkylenebis(bromoacetamide) and N,N′-alkylenebis(iodoacetamide) (wherein, in either case, the alkylene radical has 2—15 carbon atoms); the dihaloketone compounds 1,3-dibromoacetone and 1,3-diiodoacetone; and the halocarbonylmaleimide compounds N-($\alpha$-bromoacetoxymethyl)maleimide and N-($\alpha$-iodoacetoxymethyl)maleimide.

The antitumor protein hybrid of the present invention can be prepared according to the following methods.

(1) A method wherein either to make a substantial fragment Fab which has at least one S-sulfo radical in the fragment reacts with a substantial fragment A which has at least one thiol radical in the fragment or a substantial fragment Fab which has at least one thiol radical in the fragment reacts with a substantial fragment A which has at least one S-sulfo radical in the fragment.

In these methods, it is preferable to use a ratio of 0.3 to 3 moles of fragment A to 1 mole of fragment Fab. The reaction can be conducted by mixing fragment Fab and fragment A in a buffer solution whose pH is in the range of 6 to 10 to make a total protein concentration of 0.5 to 100 mg/ml (more preferably 1 to 20 mg/ml) and leaving the mixture at 0 to 60°C or dialyzing the reaction mixture against a buffer solution having the same pH value as the reaction mixture. The reaction time generally extends over a period of four hours to three days, depending upon the scale and conditions of the reaction. The separation of the hybrid thus composed of fragment Fab and fragment A from the reaction mixture and its purification can be carried out according to usual procedures, for instance, by dialysis or column chromatography of molecular sieve effect.

The method mentioned above allows the reaction to proceed smoothly under very moderate conditions to give a highly purified hybrid. The method also has the merit of permitting the selective formation of a hybrid composed of fragment Fab and fragment A (as opposed to the formation of a hybrid between fragments Fab themselves or between fragments A themselves coupled by the disulfide bond).

(2) A method for binding a substantial fragment Fab which has at least one thiol radical in the fragment and a substantial fragment A which has at least one thiol radical in the fragment with the use of any of the aforementioned cross-linking agents.

In the above method, the reaction can be conducted by bringing all three of fragment Fab, cross-linking agent and fragment A into contact with each other simultaneously; however, it is preferable to carry out the preparation of the hybrid by making fragment A react with the reaction product obtained by first allowing fragment Fab to react with the cross-linking agent or by making fragment Fab react with the reaction product obtained by first allowing fragment A to react with the cross-linking agent. In the former case, it is preferable to use 0.8 to 6 moles of the cross-linking agent and fragment A respectively to 1 mole of fragment Fab. In the latter case, it is preferable to use 0.8 to 3 moles of the cross-linking agent and 0.2 to 3 moles of fragment Fab to 1 mole of fragment A. The reaction is started at 0 to 60°C with stirring with the addition of the cross-linking agent dissolved in a small amount of solvent such as N,N-dimethylformamide, dimethyl sulfoxide, 1,2-dimethoxyethane, methanol, ethanol,

acetone, etc. to a solution of fragment Fab or fragment A buffered at a pH of 6 to 10 (the protein concentration being preferably controlled to 0.5 to 100 mg/ml, or more preferably to 1 to 20 mg/ml). After the removal of the cross-linking agent left unreacted by means of dialysis or column chromatography of molecular sieve effect, another fragment solution buffered at a pH of 6 to 10 (the preferable ranges of protein concentration being the same as mentioned above) is added to carry out the reaction at 0 to 60°C. The separation, and purification as well, of the thus obtained hybrid of fragment Fab and fragment A from the reaction mixture can be effected according to usually adopted methods such as column chromatography of molecular sieve effect.

(3) A method in which a substantial fragment Fab of the antitumor immunoglobulin which has at least one thiol radical in the fragment and a substantial fragment A of the diphtheria toxin which has at least one thiol radical in the fragment are subjected to an oxidative reaction in a state of coexistence with each other in order to have them both bound by the disulfide bond. As for the oxidative reaction, any of the following, i.e. an air oxidation method, a method using o-iodobenzoic acid and a method in which oxidation is effected in an o-phenanthroline/cupric sulfate system, may be adopted.

(4) A method in which either a substantial fragment Fab or a substantial fragment A is first made to react with Ellman's reagent (5,5'-dithiobis(2-nitrobenzoic acid), 2,2'-dipyridyldisulfide, 4,4'-dipyridyldisulfide or tetrathionate and the reaction product thus obtained is then made to react with the other of the above fragments.

In the present invention, (1) and (2) of the abovementioned methods are especially preferable.

The antitumor protein hybrid of the present invention consists of a moiety comprising a substantial fragment A which demonstrates toxicity against tumor cells and a moiety comprising a substantial fragment Fab which specifically recognizes a tumor cell and works as a carrier to guide said fragment A to the tumor cell and to have fragment A taken into the cell as well. This hybrid has excellent characteristics mentioned below.

(1) Since the hybrid of the present invention does not contain the Fc part of the immunoglobulin, nonspecific binding to Fc recepters on the cell membrane with the Fc part is avoided and this fact allows the antibody activity or performance of the fragment Fab to selectively couple to the antibody to predominate.

(2) It is known that, when a xenogeneic immunoglobulin is used, it is the Fc part that has the strongest antigenicity. In the case of the hybrid according to the present invention, since it does not contain the Fc part of the immunoglobulin, the antigenicity of the xenogeneic immunoglobulin is reduced remarkably.

(3) It is known that, in the case of diphtheria toxin, it is the fragment B that has the nature to couple to the receptor of cells (normal cells and tumor cells) and that the fragment A can be taken into the cell by means of the coupling of the fragment B to the cell membrane to demonstrate cytotoxicity. However, since the hybrid of the present invention does not contain the fragment B, the hybrid of the present invention does not demonstrate cytotoxicity to normal cells. Furthermore, since it does not contain the fragment B, the antigenicity of the diphtheria toxin is also reduced.

(4) The hybrid of the present invention has a moiety comprising a substantial fragment Fab obtained from the antitumor immunoglobulin and this moiety specifically recognizes a tumor cell and makes the tumor cell take in specifically the moiety comprising the substantial fragment A of the diphtheria toxin. The fragment A thus taken in demonstrates a remarkable cytotoxicity to the tumor cell.

The present invention is described in detail by the following examples. The measurement of the ADP-ribose transferase activity on EF-2 and the measurement of the concentration of fragment Fab' according to the single radial immunodiffusion referred to in the examples were made according to the method mentioned below.

(1) ADP-ribose transferase activity on EF-2

EF-2 was obtained from the homogenate of the rat liver and was used after partially refined by centrifugation (12000×g, supernatant fraction after twenty minutes), ammonium sulfate fractionation (supernatant of 40% saturation and precipitate of 60% saturation) and DEAE Sephadex column chromatography (equilibrated with 10 mM (m mole/l) Tris · HCl—50 mM potassium chloride, and 1 mM aqueous solution of 2-mercaptoethanol (pH 7.6)).

80 p mole of EF-2, 45 p mole of $^{14}C$ labeled $NAD^+$(6550 cpm) and 30 $\mu$l of the test specimen (prepared by diluting each fraction of column chromatography 10 to 100 times) were added to 0.1 ml of aqueous solution (pH 7.6) of 0.1 M Tris · HCl—20 mM dithiothreitol—2 mM ethylenediaminetetraacetic acid and made to react at 37°C for ten minutes. Immediately after the reaction was over, 0.5 ml of 5% trichloroacetic acid was added while cooling with an ice-bath and the precipitated substance (insoluble in acid) was collected on a 0.45 $\mu$ Millipore filter. The radioactivity of thus obtained precipitate (insoluble in acid) was measured with a liquid scintillation counter to determine the function of the test specimen to promote the undermentioned reaction or ADP-ribose transferase activity on EF-2 (the strength of the radioactivity of the precipitate is proportional to the amount of deactivated EF-2) by means of the calibration curve prepared beforehand:

$$EF\text{-}2 + NAD^+ \rightarrow (ADP\ ribose)\text{-}(EF\text{-}2) + nicotinamide + H^+$$

||

Insoluble in acid

(2) Concentration of fragment Fab' (single radial immunodiffusion)

Dithiothreitol was added to 50 μl of the respective fractions obtained from Sephadex column chromatography in such an amount as to give a concentration of 20 mM, the reductive reaction was carried on at 37°C for 30 minutes (to have the disulfide bond cleaved into two thiol radicals) and the reaction was conducted at 20°C for 30 minutes after iodoacetamide was added in such an amount as to give a concentration of 50 mM (the thiol radicals were blocked). The test specimen solution thus pretreated was placed in a well on agar which contained goat anti-rabbit IgG serum and was left standing for two days. The diameter of the ring of precipitate formed as a result of the antigen-antibody reaction was measured to determine the concentration of the fragment Fab' by means of the calibration curve prepared beforehand. However, in Examples 3 to 9, no pretreatment was made and 50 μl of the respective fractions were used as they were as test specimens (in this case, it is hard to obtain precise values quantitatively but qualitative data can be obtained satisfactorily).

Example 1

(a) Preparation of fragment Fab' of antitumor immunoglobulin

Mouse leukemia L 1210 cells transplanted successively on DBA/2Cr mice were taken out of the ascites of a DBA/2Cr mouse. An emulsion prepared from about $10^6$ of those cells and Freund's complete adjuvant (immuno adjuvant) was intravenously injected into a rabbit. After that, $10^6$ L 1210 cells, together with the adjuvant, were subcutaneously injected three times at one-week intervals, and the rabbits were bled seven days and ten days after the day of final administration. The blood samples thus obtained were mixed and serum was separated from the mixed blood and was inactivated at 56°C for 30 minutes. 200 ml of saturated aqueous ammonium sulfate solution was added to 200 ml of thus obtained serum (anti L 1210 serum) at 4°C and the precipitate (anti L 1210 immunoglobulin) was separated by means of centrifugation. The precipitate thus obtained was dissolved in 50 ml of 0.01 M phosphate buffer solution (pH 7.6) and was sufficiently purified by dialysis against the same buffer solution to obtain a solution of anti-L 1210 immunoglobulin. The solution was subjected to DEAE cellulose column chromatography (column size 3 cm×94 cm) equilibrated with the same phosphate acid buffer solution to obtain a solution containing IgG as an unadsorbed fraction. 24 mg of pepsin was added to 40 ml of anti-L 1210 IgG solution (30 mg/ml) obtained by dialyzing thus obtained solution against 0.1 M acetate buffer (pH 4.5). The pepsin digestion was carried out at 37°C for about 18 hours and the digestion product was subjected to Sephadex G200 column chromatography (column size 3.5 cm×140 cm) over saline to take out protein eluted at molecular weight of about 100,000. It was confirmed that this was purely a dimer of fragment Fab' by means of electrophoresis with sodium dodecyl sulfate. 0.04 ml of 100 mM 2-mercaptoethanol was added to 2.0 ml of a solution (7.3 mg/ml) of the dimer of fragment Fab' obtained by dialysing isotonic sodium chloride solution of the dimer of fragment Fab' ($F(ab')_2$) thus obtained against an aqueous solution (pH 8.3) of 0.01 M Tris · HCl—0.14 M sodium chloride—2 mM ethylenediaminetetraacetic acid. The reduction with 2-mercaptoethanol was carried out at 37°C for one hour and then substances of low molecular weight were removed by dialyzing against saline to obtain a fragment Fab' solution of anti-L 1210 immunoglobulin having a thiol radical (or having one thiol radical arising from one disulfide bond at the hinge part).

(b) Preparation of fragment A of diphtheria toxin

0.15 ml of a trypsin solution having the concentration of 0.1 mg/ml was added to 18.5 ml of an aqueous solution (pH 8.3) of 0.05 M Tris · HCl—2 mM ethylenediaminetetraacetic acid containing 210 mg of diphtheria toxin, and the digestion was carried out at 25°C for 50 minutes. After that 0.3 ml of a soybean trypsin inhibitor solution having the concentration of 0.5 mg/ml was added thereto to stop the reaction. Urea (final concentration 6 M), sodium sulfate (final concentration 0.168 M) and sodium tetrathionate (final concentration 0.042 M) were added to the obtained digestion product and the mixture was subjected to S-sulfonating decomposition at 37°C for two hours. The resulting reaction solution was put to Sephadex G 150 column chromatography (column size 3.5 cm×112 cm) over a buffer solution (pH 5.3) of 6 M urea—0.03 M acetic acid and only the fractions of fragment A which came out later was taken. These fractions were dialyzed against distilled water to give a pure fragment A solution (having one S-sulfo radical).

(c) Preparation of antitumor protein hybrid

2.5 ml of an aqueous solution containing 7.3 mg of fragment Fab' (having one thiol radical) of anti-L 1210 immunoglobulin IgG obtained according to the aforementioned (a) and 4.0 mg of fragment A (having one S-sulfo radical) of diphtheria toxin obtained according to the aforementioned (b) was prepared. This solution was dialyzed at 4°C for three days against 1 l of aqueous solution (pH 9.15) of 0.05 M glycine buffer—0.10 M sodium chloride—2 mM ethylenediaminetetraacetic acid to effect the reaction to couple the fragment Fab' and the fragment A. The obtained product was subjected to

Sephadex G150 column chromatography (column size 1.6 cm×93 cm) on an aqueous solution (pH 7.0) of 5 mM phosphate buffer—0.154 M sodium chloride to give 60 fractions of 2.1 ml each. The absorbance at 280 m$\mu$ was measured for each fraction to give the concentration of protein and the obtained result is shown by a solid line to indicate the pattern of protein elution in Fig. 3. As clearly shown in Fig. 3, there were four peaks in the pattern of protein elution and they were named peak 1, peak 2, peak 3, peak 4 in order of decreasing molecular weight.

The ADP-ribose transferase activity on EF-2 of the fractions corresponding to the respective peaks were measured and the results are shown by a broken line in Fig. 3. The measurement of the antigen-antibody reaction (radial immunodiffusion) with goat anti-rabbit IgG of the fractions corresponding to the respective peaks was made to know the concentration of fragment Fab′ contained in fractions corresponding to the respective peaks and the results are shown by a dotted-broken line in Fig. 3.

Fig. 3 leads to the following assumption. The protein (having the largest molecular weight) corresponding to peak 1 has no ADP-ribose transferase activity on EF-2 and contains only fragment Fab′, so it is assumed to be dimer of fragment Fab′. The protein corresponding to peak 2 has the transferase activity and also contains fragment Fab′ leading to an assumption that it is a reaction product of fragment Fab′ and fragment A. The protein corresponding to peak 3 is a mixture of protein comprising fragment Fab′ only and protein which does not contain fragment Fab′ but has the transferase activity, therefore this protein is assumed to be a mixture of fragment Fab′ and the dimer of fragment A. The protein corresponding to peak 4 (having the smallest molecular weight) does not contain fragment Fab′ but has the transferase activity, therefore it is assumed to be fragment A.

The above assumption was supported by the results of measurement of rate of movement obtained in the sodium dodecyl sulfate electrophoresis conducted on the dimer of fragment Fab′, ($F(ab')_2$), the protein corresponding to peak 2 (the 37th or 38th fraction), the dimer of fragment A, fragment Fab′, and fragment A. In Fig. 4, the arrangement of $F(ab')_2$, the protein of peak 2, the dimer of fragment A, Fab′, and fragment A is in the order of increasing rate of movement or in the order of decreasing molecular weight, and this arrangement corresponds to the arrangement of peaks 1, 2, 3 and 4 in the order of decreasing molecular weight shown in Fig. 3.

In Fig. 4, it can be read that the protein corresponding to peak 2 has the molecular weight of about $7\times10^4$ which matches well with the molecular weight of about 70,000 obtained as the sum of the molecular weight of about 46,000 of fragment Fab′ and the molecular weight of about 24,000 of fragment A. It has therefore been confirmed that the protein corresponding to peak 2 is a protein hybrid of the present invention, comprising fragment Fab′ and fragment A linked by the disulfide bond.

(d) Measurement of cytotoxicity of antitumor protein hybrid

Part 1: An aqueous solution was prepared by mixing the 37th and the 38th fractions corresponding to the aforementioned peak 2 in such a way as to contain the protein hybrid of the present invention at the concentration of 0.84 mg/ml. The cytotoxicity of the protein hybrid of the present invention against mouse leukemia cells L 1210 was measured with the use of this solution.

A culture fluid was prepared by suspending $5\times10^4$ of L 1210 cells in the medium of RPMI 1640 (containing 10% fetal calf serum and 20 $\mu$M 2-mercaptoethanol and also containing kanamycin sulfate at the concentration of 100 $\mu$g/ml). The same culture fluid was prepared in five separate lots and the mixture was incubated at 37°C in an atmosphere of 5% $CO_2$ for 42 hours with one lot untreated, another one lot treated with diphtheria toxin, and the remaining three lots treated with the protein hybrid of the present invention prepared according to the preceding (c).

After the culture was over, the cells of the respective lots of culture fluid were subjected to Trypan Blue dyeing to stain the dead cells in order to determine the number of viable cells under the microscope. The result is shown in Table 1.

TABLE 1

Cytotoxicity of antitumor protein hybrid against L 1210

| | Number of viable L 1210 cells after 42-hour incubation (%) |
|---|---|
| Not treated | $8\times10^5$ (100) |
| Diphtheria toxin, 40 $\mu$g/ml | $5\times10^5$ (63) |
| Antitumor protein hybrid, 0.5 $\mu$g/ml | $0.5\times10^5$ (6.3) |
| do., 5 $\mu$g/ml | $0.03\times10^5$ (0.4) |
| do., 50 $\mu$g/ml | $<0.01\times10^5$ (<0.1) |

Table 1 has made it clear that the initial $5\times10^4$ cells increased remarkably to 5 to $8\times10^5$ when the culture fluid was untreated or treated with diphtheria toxin, while the number of viable cells was

remarkably reduced when the culture fluid was treated with the antitumor protein hybrid of the present invention (no measurement is possible for the cells less than 1000). Incidentally, it is known that mouse cells have no susceptibility to diphtheria toxin.

Part 2: Cytotoxicity of the respective samples were examined in the same way as Part 1 of Measurement of cytotoxicity of antitumor protein hybrid mentioned above by cultivating $2\times10^4$ of mouse leukemia L-1210 cells for 48 hours in the medium RPMI 1640 comprising 10% fetal calf serum, 20 mM 2-mercaptoethanol and kanamycin sulfate at the concentration of 100 μg/ml with the addition of various samples shown in Table 2. For comparison's sake, L 1210 cells were cultivated in a system to which no samples were added (untreated) and the number of the viable cells were examined after 42 hours. The results are shown in Table 2.

TABLE 2

| | Concentration μg/ml | The number of viable L 1210 after 42-hour incubation (%) |
|---|---|---|
| Untreated | — | $3.07\times10^5$ (100) |
| Diphtheria toxin | 40 | $1.71\times10^5$ (56) |
| Fab' | 34 | $1.07\times10^5$ (35) |
| Fragment A | 18 | $1.50\times10^5$ (49) |
| Fab'+fragment A | 51 | $1.43\times10^5$ (47) |
| (molar ratio 1:1) | 5.6 | $2.00\times10^5$ (65) |
| Antitumor protein hybrid | 51 | $<0.01\times10^5$ (<0.3) |
| " | 5.1 | $<0.01\times10^5$ (<0.3) |
| " | 0.56 | $0.17\times10^5$ (5.5) |
| " | 0.056 | $1.60\times10^5$ (52) |

In the case where the antitumor protein hybrid of the present invention was added, even to such a low concentration as 0.56 μg/ml, the number of the viable cells at the time of measurement was $1.7\times10^4$ (5.5% as compared with the control), and when the concentration of the added hybrid was 5.1 μg/ml, the number of the viable cells was less than $10^3$ (counting is impossible when the number is less than $10^3$) (less than 0.3% as compared with the control). As Table 2 indicates clearly, such strong cytotoxity was hardly observed with either fragment Fab' or fragment A. Also the mixture of equimolar solutions of fragment Fab' and of fragment A scarcely had an influence on the number of the viable cells when compared with the control. This fact shows that the cytotoxicity of the antitumor protein hybrid does not arise from the mixed effect of fragment Fab' and fragment A.

Part 3: Three DBA/2Cr mice per group were inoculated intraperitoneally with $1\times10^4$ L 1210 cells. On the following day, a prescribed dose of the antitumor protein hybrid of the present invention prepared according to the foregoing (c) was administered intraperitoneally to examine its effect on the prolongation of life-span. A control group of mice having no antitumor protein hybrid administered were also prepared. The results are shown in Table 3.

TABLE 3

| Dosage, μg/mouse | Period of survival (%) |
|---|---|
| 30 | 142 |
| 6 | 138 |
| 1.2 | 125 |
| Control (not administered)* | 100 |

*All the mice of the control group died in 8 days.

It is clearly manifested in Table 3 that the mice, when administered with 1.2, 6, and 30 μg/mouse of the antitumor protein hybrid, had a period of survival of 125, 138, and 142%, respectively, as compared with the control group, which fact confirms an appreciable effect of prolonging life.

Since anti-L 1210 immunoglobulin, which was used in the aforementioned (c) Preparation of antitumor protein hybrid, did not undergo the process of absorption with the mouse normal tissue (a process to remove the antibody which recognizes the normal cells of a mouse), it contains an antibody which recognizes normal cells of a mouse. Therefore, the toxicity exercised an influence also on the host mice and the mice had the period of survival extending only to 142% even though they were

administered with the antitumor protein hybrid. It is clear that, if it underwent the process of absorption mentioned above, the antitumor protein hybrid of the present invention would have an increased selective toxicity against tumor cells to show a greater remedial effect. If anti-L 1210 antibody is subjected to the adsorption process with tumor cells or tumor antigen, the content of tumor-specific antibody can be increased; however, since this process was not effected in this example, it is assumed that the content of tumor-specific antibody in the immunoglobulin obtained in said (a) was less than 1%. Accordingly, it is obvious that when the immunoglobulin having a high content of tumor-specific antibody is obtained according to said adsorption process and is used in the preparation of the antitumor protein hybrid, a greater remedial effect can be obtained with a smaller dosage.

Example 2

(a) Preparation of fragment Fab′ of antitumor immunoglobulin

21 mg of sodium sulfite and 13 mg of sodium tetrathionate were added to 3 ml of a saline solution containing 29 mg of the dimer of fragment Fab′ of rabbit antibody (immunoglobulin IgG) against mouse leukemia L 1210 prepared according to Example 1, (a), and after the mixture was subjected to S-sulfonative cleavage at 37°C for one hour to obtain fragment Fab′ having a S-sulfo radical, the reagents were removed by dialysis.

(b) Preparation of fragment A of diphtheria toxin

0.05 ml of 0.5 M aqueous solution of 2-mercaptoethanol was added to 1 ml of a solution of fragment A (4.8 mg/ml) obtained by dialyzing the solution of fragment A having one S-sulfo radical of diphtheria toxin prepared according to Example 1, (b) against an aqueous solution (pH 8.3) of 0.01 M Tris · HCl—0.14 M sodium chloride—2 mM ethylenediaminetetraacetic acid, and the mixture was reduced at 37°C for one hour. After that, 2-mercaptoethanol was removed by means of dialysis to obtain fragment A having one thiol radical.

(c) Preparation of antitumor protein hybrid

2 ml of an aqueous solution containing 5.8 mg of fragment Fab′ prepared in the aforementioned (a) and 3.2 mg of fragment A prepared in the preceding (b) was prepared. This solution was dialyzed at 4°C for three days against 1 l of an aqueous solution (pH 9.15) of 0.05 M glycine buffer—0.10 M sodium chloride—2 mM ethylenediaminetetraacetic acid to carry out the reaction to link both fragments. The reaction was followed by the same procedures as described in Example 1 to give a protein hybrid of the present invention, having fragment Fab′ and fragment A linked with a disulfide bond.

Example 3

(a) Preparation of antitumor protein hybrid

0.05 ml of 20 mM solution of o-phenylenedimaleimide (PDM) in N,N-dimethylformamide was added to 1 ml of an aqueous solution (pH 5.5) of 5 mM acetate buffer—0.14 M sodium chloride—1 mM ethylenediaminetetraacetic acid containing 36.8 mg of fragment Fab′ of anti-L 1210 immunoglobulin having one thiol radical obtained according to Example 1, (a), and the mixture was allowed to react at 30°C for 30 minutes. The reaction mixture was subjected to gel-filtration on Sephadex G25 (column size 0.7 cm×35 cm) with 5 mM acetic acid buffer—0.14 M sodium chloride—1 mM ethylenediaminetetraacetic acid (pH 5.5) to remove excessive reagents.

1.0 ml of an aqueous solution of fragment A (2.15 mg/ml) of diphtheria toxin having one thiol radical prepared according to Example 2, (b) and 0.2 ml of 0.3 M sodium phosphate buffer (pH 6.5) were added to 1.04 ml of solution of fragment Fab′ having the PDM residue (4.29 mg/ml) of anti-L 1210 immunoglobulin thus obtained, and the mixture was allowed to react at 4°C overnight. Then the reaction mixture was subjected to Sephadex G150 superfine column chromatography (column size 1.6 cm×93 cm) with an 0.9% aqueous solution of sodium chloride to obtain a solution containing 4.4 mg of the antitumor protein hybrid by collecting the 38th to 42nd fractions.

It was confirmed according to the following method that what was contained in those fractions was the desired substance. In order to ascertain the protein concentration of the respective fractions obtained by said column chromatography, their absorbance was measured at 280 mμ. The resulting elution pattern of protein had three peaks 1, 2 and 3 as shown in Fig. 5. The proteins at those peaks were subjected to analysis according to the Ouchterlony method using guinea pig anti-rabbit Fab′ antibody and horse anti-diphtheria toxin antibody and the results as shown in Fig. 6 were obtained. The proteins at peaks 1 and 2 formed a precipitate line with anti-rabbit Fab′ antibody and those at peaks 1 and 3 with anti-diphtheria toxin antibody. Therefore, it is clear that peak 1 contains Fab′ and fragment A of diphtheria toxin as well. Also it is known from the point of elution that the protein at peak 1 has molecular weight of about 70,000, which corresponds to the total of molecular weight of fragment Fab′ (46,000) and molecular weight of fragment A of diphtheria toxin (24,000). It may be given as a conclusion from the above results that the protein at peak 1 is the antitumor hybrid, comprising 1 molecule of fragment Fab′ and 1 molecule of fragment A of diphtheria toxin bonded by PDM, of the present invention. Incidentally, those proteins at peaks 2 and 3 are respectively fragment Fab′ and fragment A of diphtheria toxin remaining not reacted, either modified or not modified by PDM.

(b) Measurement of cytotoxicity of antitumor protein hybrid

Mouse leukemia L 1210 cells ($3\times10^4$) were cultivated in an atmosphere of 5% carbon dioxide at 37°C for 1, 2, 3, and 4 days respectively in 1-ml RPMI media each containing 10% fetal calf serum and 20 μM 2-mercaptoethanol, also containing kanamycin sulfate at a concentration of 100 μg/ml and the added antitumor protein hybrid prepared in the preceding (a) at the final concentration of 30, 6, and 1.2 μg/ml, respectively. For comparison's sake, L 1210 cells were cultivated in a medium with fragment Fab' and fragment A of diphtheria toxin added at the concentration of 20 μg/ml and 10 μg/ml respectively (at molar ratio of 1:1) in the place of the antitumor protein hybrid. As for the control, L 1210 cells were cultivated in the system to which none of the antitumor protein hybrid, fragment Fab', and fragment A of diphtheria toxin was added. After the cultivation was over, uniform suspensions of cells were prepared with the use of a pipette and then dyed with 0.3% Trypan Blue-phosphate buffered saline, and the number of viable cells was counted under the microscope. The results are shown in Fig. 7.

It is clear from Fig. 7 that the antitumor protein hybrid shows a remarkable effect of cytotoxicity of the concentration of 30 and 6 μg/ml. The effect was especially remarkable from the second day of cultivation. On the other hand, no cytotoxicity was observed at all with the mixed solution of fragment Fab' and fragment A of diphtheria toxin mixed at the molar ratio of 1:1 (in which both were not bonded). It is therefore understood that it is necessary for both fragments to be bonded into a hybrid with a covalent bond for the manifestation of toxicity.

Example 4

Using the procedure of Example 3, antitumor protein hybrid was obtained in which fragment Fab' and fragment A were linked by a cross-linking agent via the respective sulfur atoms, N,N'-(1,3-phenylene)dimaleimide being used as the cross-linking agent in place of PDM which was used in Example 3.

Example 5

Using the procedure of Example 3, antitumor protein hybrid was obtained in which fragment Fab' and fragment A were linked by a cross-linking agent via the respective sulfur atoms, 4,4'-bis(maleoylamino)azobenzene being used as the cross-linking in place of PDM which was used in Example 3.

Example 6

Fragment Fab' of anti-L 1210 immunoglobulin IgG having one thiol radical obtained according to Example 1, (a), was dissolved in a mixed solution consisting of 3 parts by volume of 0.1 M sodium phosphate buffer (pH 6.0) and 1 part by volume of N,N-dimethylformamide at a concentration of 7 mg/ml to prepare a solution of fragment Fab'. Another solution was prepared by dissolving a cross-linking agent, N,N'-ethylenebis(iodoacetamide), in N,N-dimethylformamide at a concentration of 6 mg/ml.

The reaction was carried out at room temperature for one hour by adding 0.1 ml of the N,N'-ethylenebis(iodoacetamide) solution dropwise to 1.0 ml of the fragment Fab' solution, to which reaction mixture 0.05 ml of 0.07 M aqueous solution of 2-mercaptoethylamine was added. The mixture was left standing at room temperature for one hour. The obtained mixed solution was purified by column chromatography on Sephadex G25 equilibrated with 0.1 M sodium phosphate buffer (pH 6.0) to give a solution of fragment Fab' having an N,N'-ethylenebis(iodoacetamide) residue.

The fragment A of diphtheria toxin having one thiol radical prepared according to Example 2, (b), was added to the solution of fragment Fab' having N,N'-ethylenebis(iodoacetamide) residue obtained as mentioned above to make the molar ratio of fragment Fab' having N,N'-ethylenebis(iodoacetamide) residue to fragment A 1:2 and mixed. After that, the procedures were followed as in the case of Example 3 to give protein hybrid of the present invention in which fragment Fab' and fragment A were linked by a cross-linking agent, N,N'-ethylenebis(iodoacetamide), through the medium of the respective sulfur atoms. This protein hybrid had almost the same remarkable cytotoxic activity against L 1210 as the one according to Example 1.

Example 7

Using the procedure of Example 6, antitumor protein hybrid was obtained in which fragment Fab' and fragment A were linked by a cross-linking agent through the medium of the respective sulfur atoms, N,N'-hexamethylenebis(iodoacetamide) being used as the cross linking agent in place of N,N'-ethylenebis(iodoacetamide) which was used in Example 6.

Example 8

Using the procedure of Example 6, antitumor protein hybrid was obtained, in which fragment Fab' and fragment A were linked by a cross-linking agent through the medium of the respective sulfur atoms N,N'-undecamethylenebis(iodoacetamide) being used as the crosslinking agent in place of N,N'-ethylenebis(iodoacetamide) which was used in Example 6.

Example 9

Example 6, antitumor protein hybrid was obtained, in which fragment Fab′ and fragment A were linked by a cross-linking agent through the medium of the respective sulfur atoms, bis(N-maleimidomethyl)ether being used as the cross-linking agent in place of N,N′-ethylenebis(iodoacetamide) which was used in Example 6.

**Claims**

1. Antitumor protein hybrid which is expressed by the following formula (I):

$$Fab\text{-}(S_1\text{—}(X)_n\text{—}S_2\text{—}FA)_m \qquad (I)$$

where

Fab indicates a moiety which is a fragment having antibody activity arising from the Fab part of an anti-tumor immunoglobulin;

FA indicates a moiety which is a fragment of a diphtheria toxin which satisfies the characteristics of fragment A of a diphtheria toxin;

X indicates a divalent organic radical derived from N,N′-(1,3-phenylene)-dimaleimide, N,N′-(1,2-phenylene)dimaleimide, N,N′-(1,4-phenylene)dimaleimide, 4,4′-bis(maleoylamino)azobenzene, bis(N-maleimidomethyl)ether, N,N′-alkylenebis(bromoacetamide) or N,N′-alkylenebis(iodoacetamide) (wherein the alkylene radical has 2—15 carbon atoms), 1,3-dibromoacetone, 1,3-diiodoacetone, N-($\alpha$-bromoacetoxymethyl)maleimide or N-($\alpha$-iodoacetoxymethyl)maleimide;

$S_1$ and $S_2$ are both sulfur atoms, $S_1$ indicating a sulfur atom arising from the disulfide bond (—S—S— bond) in an immunoglobulin and $S_2$ a sulfur atom arising from the disulfide bond in a diphtheria toxin;

n stands for 0 or 1; and

m stands for 1 or 2.

2. Antitumor protein hybrid according to claim 1 which is expressed by the following formula (II):

$$Fab\text{-}(S_1\text{—}S_2\text{—}FA)_m \qquad (II)$$

where

Fab, FA, m, $S_1$ and $S_2$ have the meanings given in claim 1.

3. Antitumor protein hybrid according to claim 1 or claim 2, wherein FA is the fragment A of a diphtheria toxin.

4. Antitumor protein hybrid according to claim 1, 2 or 3, wherein Fab is the fragment Fab of an antitumor immunoglobulin.

5. Antitumor protein hybrid according to claim 1, 2 or 3, wherein Fab is the fragment Fab′ of an antitumor immunoglobulin.

6. A process for preparing an antitumor protein hybrid which is expressed by the following formula (II)

$$Fab\text{-}(S_1\text{—}S_2\text{—}FA)_m \qquad (II)$$

where

Fab indicates a moiety which is a fragment having antibody activity arising from the Fab part of an antitumor immunoglobulin;

FA indicates a moiety which is a fragment of a diphtheria toxin which satisfies the characteristics of fragment A of a diphtheria toxin;

$S_1$ and $S_2$ are both sulfur atoms, $S_1$ indicating a sulfur atom arising from the disulfide bond (—S—S— bond) in an immunoglobulin and $S_2$ a sulfur atom arising from the disulfide bond in a diphtheria toxin; and

m stands for 1 or 2 characterised in that a fragment of an antitumor immunoglobulin, which fragment has antibody activity arising from the Fab part of the said immunoglobulin and has at least one S-sulfo radical, is reacted with a fragment of a diphtheria toxin, which fragment satisfies the requirements of the fragment A of a diphtheria toxin and has at least one thiol radical.

7. A process for preparing an antitumor protein hybrid which is expressed by the following formula (II)

$$Fab\text{-}(S_1\text{—}S_2\text{—}FA)_m \qquad (II)$$

(where Fab, FA, $S_1$, $S_2$ and m have the meanings given in claim 6), characterised in that a fragment of an antitumor immunoglobulin, which fragment has antibody activity arising from the Fab part of the said immunoglobulin and has at least one thiol radical, is reacted with a fragment of a diphtheria toxin, which fragment satisfies the requirements of the fragment A of a diphtheria toxin and has at least one S-sulfo radical.

8. A process for preparing an antitumor protein hybrid which is expressed by the following formula (I')

$$Fab\text{+}(S_1\text{—}X\text{—}S_2\text{—}FA)_m \qquad (I')$$

where

Fab, FA, $S_1$, $S_2$ and m have the meanings given in claim 6; and

X indicates a divalent organic radical derived from N,N'-(1,3-phenylene)dimaleimide, N,N'-(1,2-phenylene)dimaleimide, N,N'-(1,4-phenylene)dimaleimide, 4,4'-bis(maleoylamino)azobenzene, bis(N-maleimidomethyl)ether, N,N'-alkylenebis(bromoacetamide) or N,N'-alkylenebis(iodoacetamide) (wherein the alkylene radical has 2—15 carbon atoms), 1,3-dibromoacetone, 1,3-diiodoacetone, N-($\alpha$-bromoacetoxymethyl)maleimide or N-($\alpha$-iodoacetoxymethyl)maleimide) characterised in that it comprises binding a fragment of an antitumor immunoglobulin, which fragment has antibody activity arising from the Fab part of the said immunoglobulin and which has at least one thiol radical, with a fragment of a diphtheria toxin, which fragment satisfies the requirements of the fragment A of a diphtheria toxin and has at least one thiol radical, with the use of a cross-linking agent which comprises a group X (as defined above) and has at least two functional groups capable of reacting with a thiol radical.

9. A process for preparing an antitumor protein hybrid which is expressed by the general formula (II) as given in claim 6, characterised in that either a fragment of an antitumor immunoglobulin, which fragment has antibody activity arising from the Fab part of the said immunoglobulin, or a fragment of a diphtheria toxin, which fragment satisfies the requirements of the fragment A of a diphtheria toxin, is reacted with 5,5'-dithiobis(2-nitrobenzoic acid), 2,2'-dipyridyldisulfide, 4,4'-dipyridyldisulfide or tetrathionate and the reaction product so obtained is reacted with the other of the said fragments.

10. A process for preparing an antitumor protein hybrid which is expressed by the general formula (II) as given in claim 6, characterised in that a fragment of an antitumor immunoglobulin, which fragment has antibody activity arising from the Fab part of the said immunoglobulin and has at least one thiol radical, and a fragment of a diphtheria toxin, which fragment satisfies the requirements of the fragment A of a diphtheria toxin and has at least one thiol radical, are together subjected to an oxidative reaction whereby the said fragments are bound by a disulfide bond.

## Revendications

1. Hybride protéinique antitumoral, représenté par la formule (I):

$$Fab\text{+}(S_1\text{—}(X)_n\text{—}S_2\text{—}FA)_m \qquad (I)$$

où Fab indique un fragment ayant une activité d'anticorps due à la partie Fab d'une immunoglobuline antitumorale,

FA indique un fragment d'une toxine diphtérique qui répond aux caractéristiques du fragment A d'une toxine diphtérique;

X indique un radical organique bivalent dérivé du N,N'-(phénylène-1,3)dimaléimide, du N,N'-(phénylène-1,2)dimaléimide; du N,N'-(phénylène-1,4)dimaléimide, du bis (maléoylamino)-4,4' azobenzène, du bis(N-maléimidométhyl)éther, du N,N'-alkylènebis (bromacétamide) ou du N,N'-alkylènebis(iodacétamide) (où le radical alkylène a 2 à 15 atomes de carbone), de la dibromo-1,3 acétone, de la diiodo-1,3 acétone, du N-($\alpha$-bromoacétoxyméthyl)maléimide ou du N-($\alpha$-iodoacétoxyméthyl)maléimide;

$S_1$ et $S_2$ sont tous deux des atomes de soufre, $S_1$ indiquant un atome de soufre provenant d'une liaison disulfure (liaison —S—S—) d'une immunoglobuline et $S_2$ un atome de soufre provenant de la liaison bisulfure d'une toxine diphtérique;

n vaut 0 ou 1 et m vaut 1 ou 2.

2. Hybride de protéine antitumoral selon la revendication 1, caractérisé en ce qu'il est représenté par la formule suivante (II):

$$Fab\text{+}(S_1\text{—}S_2\text{—}FA)_m \qquad (II)$$

où Fab, FA, m, $S_1$ et $S_2$ ont les significations données dans la revendication 1.

3. Hybride de protéine antitumoral selon l'une quelconque des revendications 1 ou 2, caractérisé en ce que FA est le fragment A d'une toxine diphtérique.

4. Hybride protéinique antitumoral selon l'une quelconque des revendications 1 à 3, caractérisé en ce que Fab est le fragment Fab d'une immunoglobuline antitumorale.

5. Hybride de protéine antitumoral selon l'une quelconque des revendications 1 à 3, caractérisé en ce que Fab est le fragment Fab' d'une immunoglobuline antitumorale.

6. Procédé de préparation d'un hybride de protéine antitumoral, représenté par la formule suivante (II)

$$Fab\{S_1—S_2—FA)_m \quad (II)$$

où

Fab indique un fragment ayant une activité d'anticorps et provenant de la partie Fab d'une immunoglobuline antitumorale,

FA indique un fragment d'une toxine diphtérique qui répond aux caractéristiques du fragment A d'une toxine diphtérique;

$S_1$ et $S_2$ sont tous les deux des atomes de soufre, $S_1$ indiquant un atome de soufre provenant de la liaison disulfure (liaison —S—S—) d'une immunoglobuline et $S_2$ un atome de soufre provenant de la liaison disulfure d'une toxine diphtérique; et

m vaut 1 ou 2, caractérisé en ce qu'on fait réagir un fragment d'une immunoglobuline antitumorale, fragment qui possède une activité d'anticorps provenant de la partie Fab de cette immunoglobuline et a au moins un radical S-sulfo, avec un fragment d'une toxine diphtérique, fragment qui a les propriétés du fragment A d'une toxine diphtérique et possède au moins un radical thiol.

7. Procédé de préparation d'un hybride protéinique antitumoral représenté par la formule suivante (II)

$$Fab\{S_1—S_2—FA)_m \quad (II)$$

où Fab, FA, $S_1$, $S_2$ et m ont les significations données dans la revendication 6, caractérisé en ce qu'on fait réagir un fragment d'une immunoglobuline antitumorale, fragment qui possède une activité d'anticorps provenant de la partie Fab de cette immunoglobuline et a au moins un radical thiol, avec un fragment d'une toxine diphtérique, fragment qui répond aux conditions du fragment A d'une toxine diphtérique et a au moins un radical S-sulfo.

8. Procédé de préparation d'un hybride protéinique antitumoral représenté par la formule suivante (I')

$$Fab\{S_1—X—S_2—FA)_m \quad (I')$$

où Fab, FA, $S_1$, $S_2$ et m ont les significations données dans la revendication 6; et X indique un radical organique bivalent dérivé du N,N'-(phénylène-1,3)dimaléimide, du N,N'-(phénylène-1,2)dimaléimide, du N,N'-(phénylène-1,4)dimaléimide, du bis(maléoylamino)-4,4'-azobenzène, du bis(N-maléimidométhyl)éther, du N,N'-alkylènebis(bromoacétamide) ou du N,N'-alkylènebis(iodoacétamide) (où le radical alkylène a 2 à 15 atomes de carbone), de la dibromo-1,3 acétone, de la diiodo-1,3 acétone, du N-($\alpha$-bromoacétoxyméthyl)maléimide ou du N-($\alpha$-iodoacétoxyméthyl)maléimide, caractérisé en ce qu'il consiste à relier un fragment d'une immunoglobuline antitumorale, fragment qui possède une activité d'anticorps provenant de la partie Fab de cette immunoglobuline et a au moins un radical thiol, à un fragment d'une toxine diphtérique, fragment qui a les propriétés nécessaires du fragment A d'une toxine diphtérique et a au moins un radical thiol, à l'aide d'un agent de pontage comprenant un groupe X (tel que défini ci-dessus) et possédant au moins deux groupes fonctionnels à même de réagir sur un radical thiol.

9. Procédé de préparation d'un hybride de protéine antitumoral représenté par la formule générale (II) telle que donnée dans la revendication 6, caractérisé en ce qu'on fait réagir un fragment d'une immunoglobuline antitumorale, fragment qui possède une activité d'anticorps provenant de la partie Fab de cette immunoglobuline, ou un fragment d'une toxine diphétrique, fragment qui a les propriétes caractéristiques du fragment A d'une toxine diphtérique, avec de l'acide dithio-5,5'bis(nitro-2 benzoïque), du bisulfure de dipyridyle-2,2', du bisulfure de dipyridyle-4,4' ou du tétrathionate, le produit ainsi obtenu étant mis à réagir sur l'autre des fragments.

10. Procédé pour la préparation d'un hybride de protéine antitumoral représenté par la formule générale (II) telle que donnée dans la revendication 6, caractérisé en ce qu'on soumet ensemble à une réaction d'oxydation par laquelle ces fragments sont reliés par une liaison disulfure, un fragment d'une immunoglobuline antitumorale, fragment qui possède une activité d'anticorps provenant de la partie Fab de cette immunoglobuline et possède au moins un radical thiol, et un fragment d'une toxine diphtérique, fragment qui a les propriétés du fragment A d'une toxine diphtérique et possède au moins un radical thiol.

**Patentansprüche**

1. Antitumorproteinhybrid der folgenden Formel (I)

$$Fab\{S_1—(X)_n—S_2—FA)_m \quad (I)$$

worin Fab für einen Anteil steht, der ein Fragment ist, welches eine Antikörperaktivität besitzt, die auf den Fab-Teil eines Antitumorimmunoglobulins zurückgeht, FA einen Anteil versinnbildlicht, der ein Fragment eines Diphtherietoxins ist, welches den Eigenschaften eines Fragments A eines Diphtherietoxins entspricht, X ein zweiwertiger organischer Rest ist, der auf N,N'-(1,3-phenylen)dimaleimid, N,N'-(1,2-phenylen)dimaleimid, N,N'-(1,4-phenylen)dimaleimid, 4,4'-bis(maleoylamino)azobenzol, bis(N-maleimidomethyl)ether, N,N'-alkylen-bis(bromacetamid) oder N,N'-alkylen-bis(jodacetamid), wobei der Alkylenrest 2 bis 15 Kohlenstoffatome aufweist, 1,3-Dibromaceton, 1,3-Dijodaceton, N-($\alpha$-bromacetoxymethyl)maleimid oder N-($\alpha$-jodacetoxymethyl)maleimid zurückgeht,

$S_1$ und $S_2$ jeweils Schwefelatome sind, wobei $S_1$ ein Schwefelatom ist, das auf die Disulfidbindung (—S—S— Bindung) in einem Immunoglobulin zurückgeht und $S_2$ ein Schwefelatom ist, das auf die Disulfidbindung in einem Diphtherietoxin zurückgeht,

n für 0 oder 1 steht und

m 1 oder 2 bedeutet.

2. Antitumorproteinhybrid nach Anspruch 1 der Formel (II)

$$Fab \leftarrow S_1—S_2—FA)_m \qquad (II)$$

worin Fab, FA, m, $S_1$ und $S_2$ die im Anspruch 1 angegebenen Bedeutungen besitzen.

3. Antitumorproteinhybrid nach Anspruch 1 oder 2, dadurch gekennzeichnet, daß FA das Fragment A eines Diphtherietoxins ist.

4. Antitumorproteinhybrid nach Anspruch 1, 2 oder 3, dadurch gekennzeichnet, daß Fab das Fragment Fab eines Antitumorimmunoglobulins ist.

5. Antitumorproteinhybrid nach Anspruch 1, 2 oder 3, dadurch gekennzeichnet, daß Fab das Fragment Fab' eines Antitumorimmunoglobulins ist.

6. Verfahren zur Herstellung eines Antitumorproteinhybrids der Formel (II)

$$Fab \leftarrow S_1—S_2—FA)_m \qquad (II)$$

worin Fab einen Anteil bedeutet, der ein Fragment ist, das eine Antikörperaktivität besitzt, die auf den Fab-Teil eines Antitumorimmunoglobulins zurückgeht, FA einen Anteil bedeutet, der ein Fragment eines Diphtherietoxins ist, das den Eigenschaften des Fragments A eines Diphtherietoxins entspricht, $S_1$ und $S_2$ jeweils Schwefelatome sind, wobei $S_1$ für ein Schwefelatom steht, das auf die Disulfidbindung (—S—S—Bindung) eines Immunoglobulins zurückgeht, und $S_2$ ein Schwefelatom ist, das auf die Disulfidbindung in einem Diphtherietoxin zurückgeht und m für 1 oder 2 steht, dadurch gekennzeichnet, daß ein Fragment eines Antitumorimmunoglobulins, das eine Antikörperaktivität besitzt, die auf den Fab-Teil des Immunoglobulins zurückgeht, und wenigstens einen S-Sulforest aufweist, mit einem Fragment eines Diphtherietoxins umgesetzt wird, das den Anforderungen des Fragments A eines Diphtherietoxins entspricht und wenigstens einen Thiolrest besitzt.

7. Verfahren zur Herstellung eines Antitumorproteinhybrids der folgenden Formel (II)

$$Fab \leftarrow S_1—S_2—FA)_m \qquad (II)$$

worin Fab, FA, $S_1$, $S_2$ und m die in Anspruch 6 angegebene Bedeutung besitzen, dadurch gekennzeichnet, daß ein Fragment eines Antitumorimmunoglobulins, das eine Antikörperaktivität besitzt, die auf den Fab-Teil des Immunoglobulins zurückgeht, und wenigstens einen Thiolrest besitzt, mit einem Fragment eines Diphtherietoxins umgesetzt wird, wobei dieses Fragment den Anforderungen des Fragments A eines Diphtherietoxins entspricht und wenigstens einen S-Sulforest besitzt.

8. Verfahren zur Herstellung eines Antitumorproteinhybrids der Formel (I')

$$Fab \leftarrow S_1—X—S_2—FA)_m \qquad (I')$$

worin Fab, Fa, $S_1$, $S_2$ und m die in Anspruch 6 angegebene Bedeutung besitzen und X für einen zweiwertigen organischen Rest steht, der auf N,N'-(1,3-phenylen)dimaleimid, N,N'-(1,2-phenylen)dimaleimid, N,N'-(1,4-phenylen)dimaleimid, 4,4'-bis(maleoylamino)azobenzol, bis(N-maleimidomethyl)ether, N,N'-alkylen-bis(bromoacetamid) oder N,N'-alkylen-bis(jodacetamid), wobei der Alkylenrest 2 bis 15 Kohlenstoffatome aufweist, 1,3-Dibromaceton, 1,3-Dijodaceton, N-($\alpha$-bromacetoxymethyl)maleimid oder N-($\alpha$-jodacetoxymethyl)maleimid zurückgeht, dadurch gekennzeichnet, daß ein Fragment eines Antitumorimmunoglobulins, das eine Antikörperaktivität besitzt, die auf den Fab-Teil des Immunoglobulins zurückgeht und wenigstens einen Thiolrest besitzt, mit einem Fragment eines Diphtherietoxins umgesetzt wird, welches den Anforderungen des Fragments A eines Diphtherietoxins entspricht und wenigstens einen Thiolrest aufweist, wobei ein Vernetzungsmittel eingesetzt wird, das eine Gruppe X gemäß vorstehender Definition aufweist und wenigstens zwei funktionelle Gruppen besitzt, die zu einer Reaktion mit einem Thiolrest befähigt sind.

9. Verfahren zur Herstellung eines Antitumorproteinhybrids der Formel (II) gemäß Anspruch 6, dadurch gekennzeichnet, daß entweder ein Fragment eines Antitumorimmunoglobulins, das eine Antikörperaktivität besitzt, die auf den Fab-Teil des Immunoglobulins zurückgeht, oder ein Fragment eines Diphtherietoxins, das den Anforderungen des Fragments A eines Diphtherietoxins entspricht, mit 5,5'-Dithio-bis(2-nitrobenzoesäure), 2,2'-Dipyridyldisulfid, 4,4'-Dipyridyldisulfid oder Tetrathionat

umgesetzt wird und das auf diese Weise erhaltene Reaktionsprodukt mit dem anderen der Fragmente zur Umsetzung gebracht wird.

10. Verfahren zur Herstellung eines Antitumorproteinhybrids der allgemeinen Formel (II) gemäß Anspruch 6, dadurch gekennzeichnet, daß ein Fragment eines Antitumorimmunoglobulins, das eine Antikörperaktivität besitzt, die auf den Fab-Teil des Immunoglobulins zurückgeht und wenigstens einen Thiolrest besitzt, und ein Fragment eines Diphtherietoxins, das den Anforderungen des Fragments A eines Diphtherietoxis entspricht und wenigstens einen Thiolrest besitzt, zusammen einer oxidativen Reaktion unterzogen werden, wobei die Fragmente über eine Disulfidbindung verknüpft werden.

FIG. 1

(a)

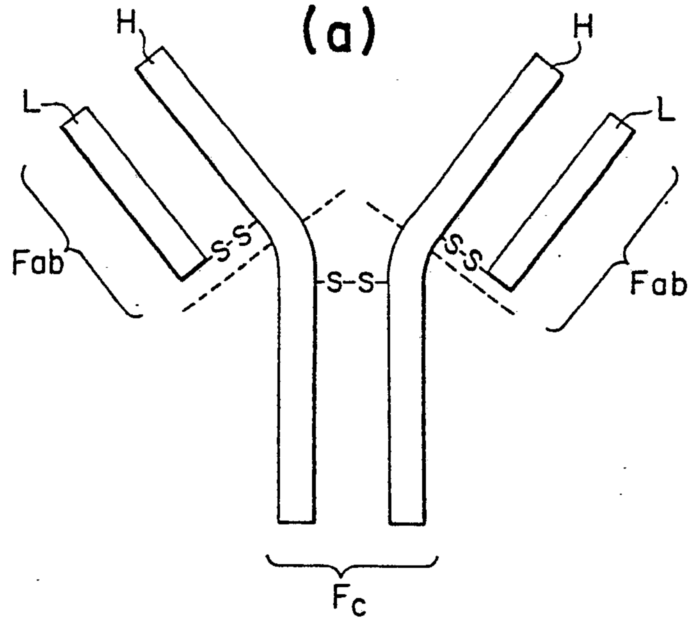


(b)

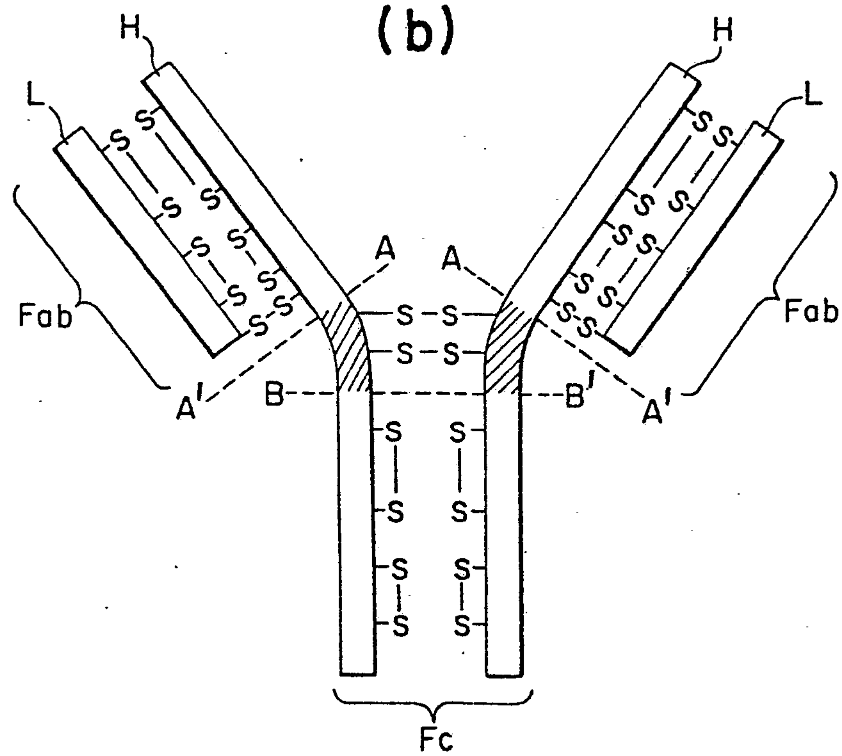

FIG. 2

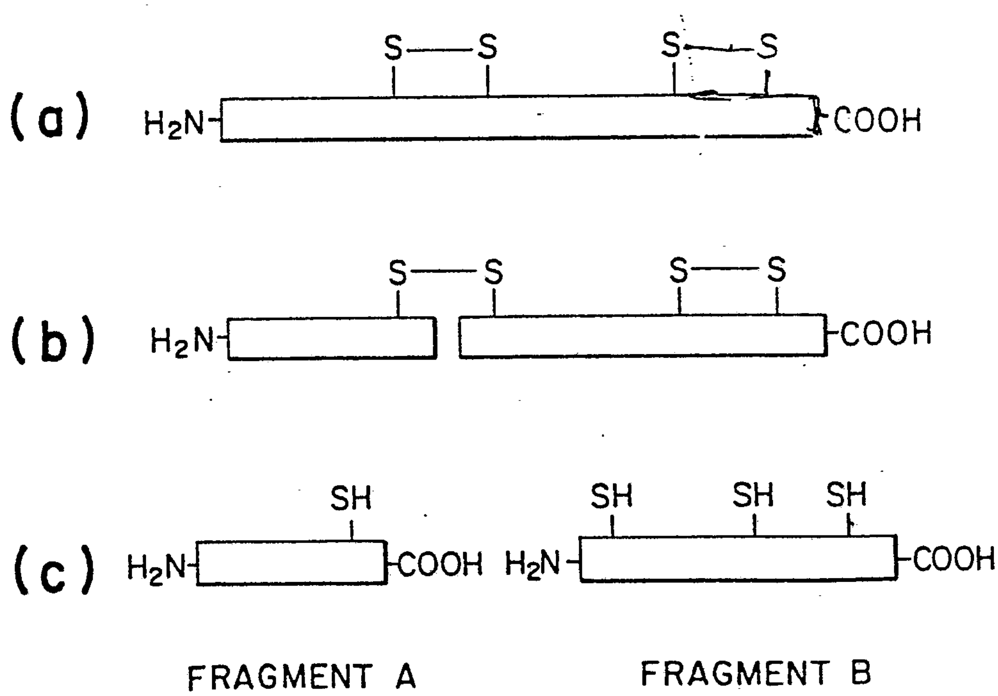
(a)
S
S
S
S
H2N
COOH
(b)
S
S
S
S
H2N
COOH
(c)
SH
H2N
COOH
H2N
SH
SH
SH
COOH
FRAGMENT A
FRAGMENT B

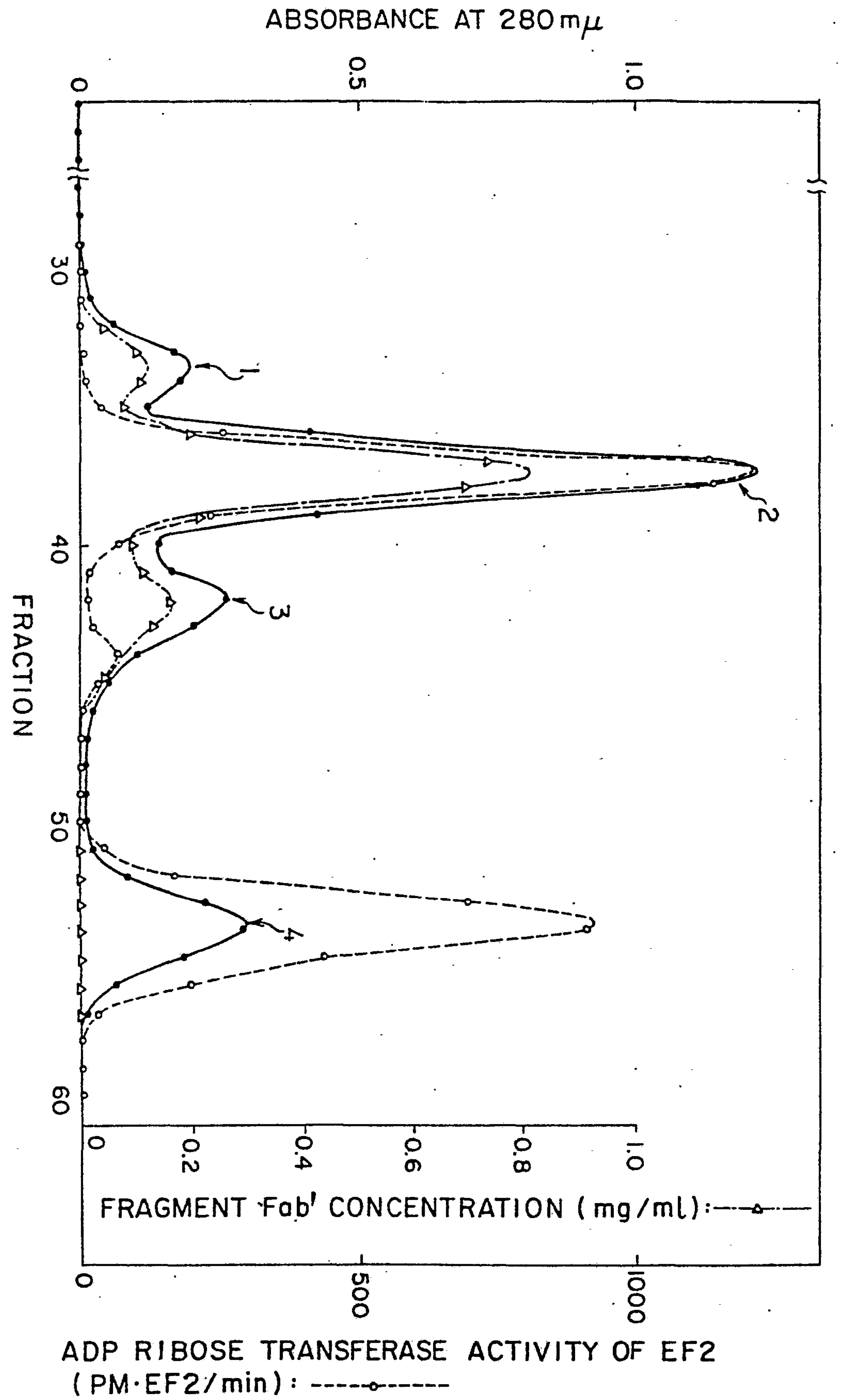
ABSORBANCE AT 280 mμ
0
0.5
1.0
30
40
50
60
FRACTION
1
2
3
4
0
0.2
0.4
0.6
0.8
1.0
FRAGMENT Fab' CONCENTRATION (mg/ml)
0
500
1000
ADP RIBOSE TRANSFERASE ACTIVITY OF EF2
(PM·EF2/min)

FIG. 3

FIG. 4

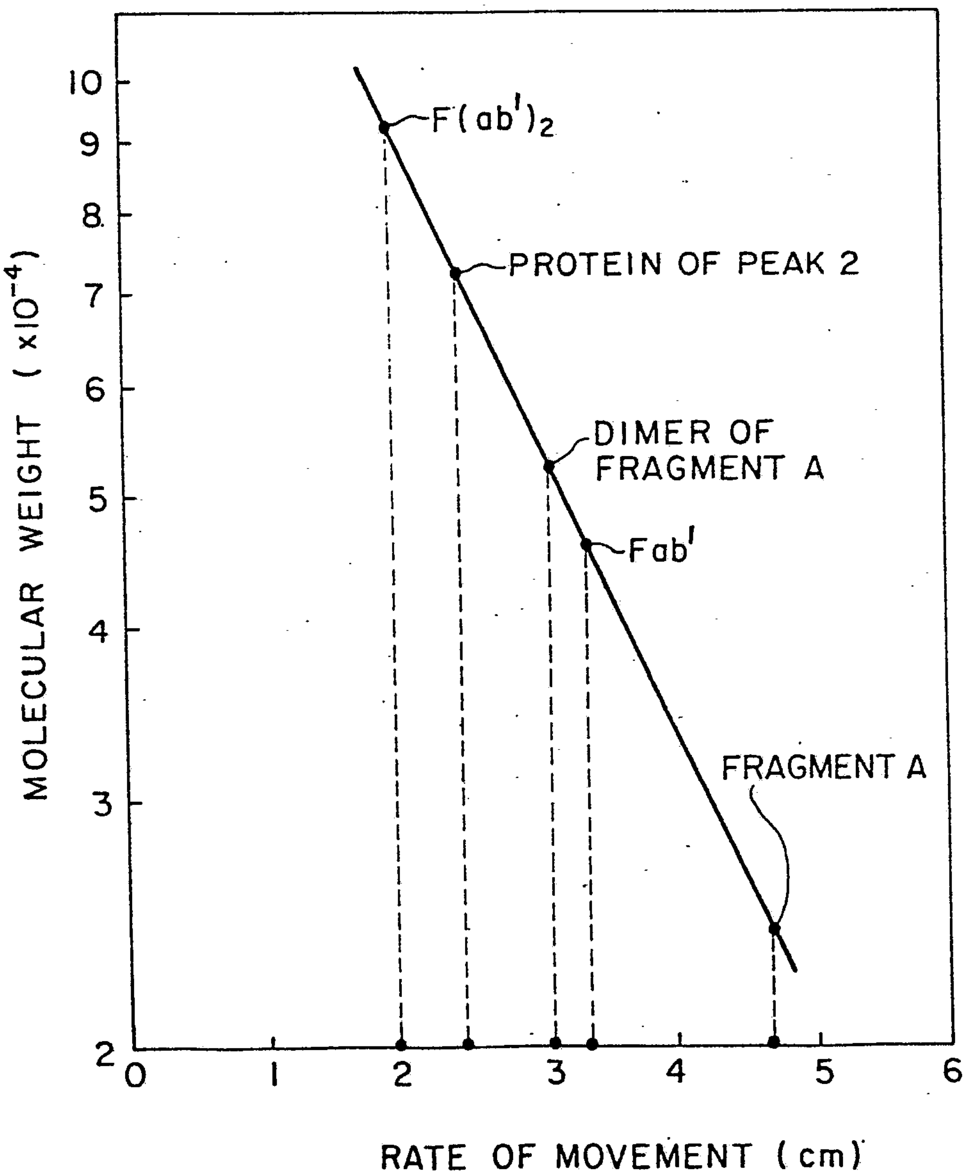
10
9
8
7
6
5
4
3
2
MOLECULAR WEIGHT (x10⁻⁴)
F(ab')2
PROTEIN OF PEAK 2
DIMER OF FRAGMENT A
Fab'
FRAGMENT A
0
1
2
3
4
5
6
RATE OF MOVEMENT (cm)

FIG. 5

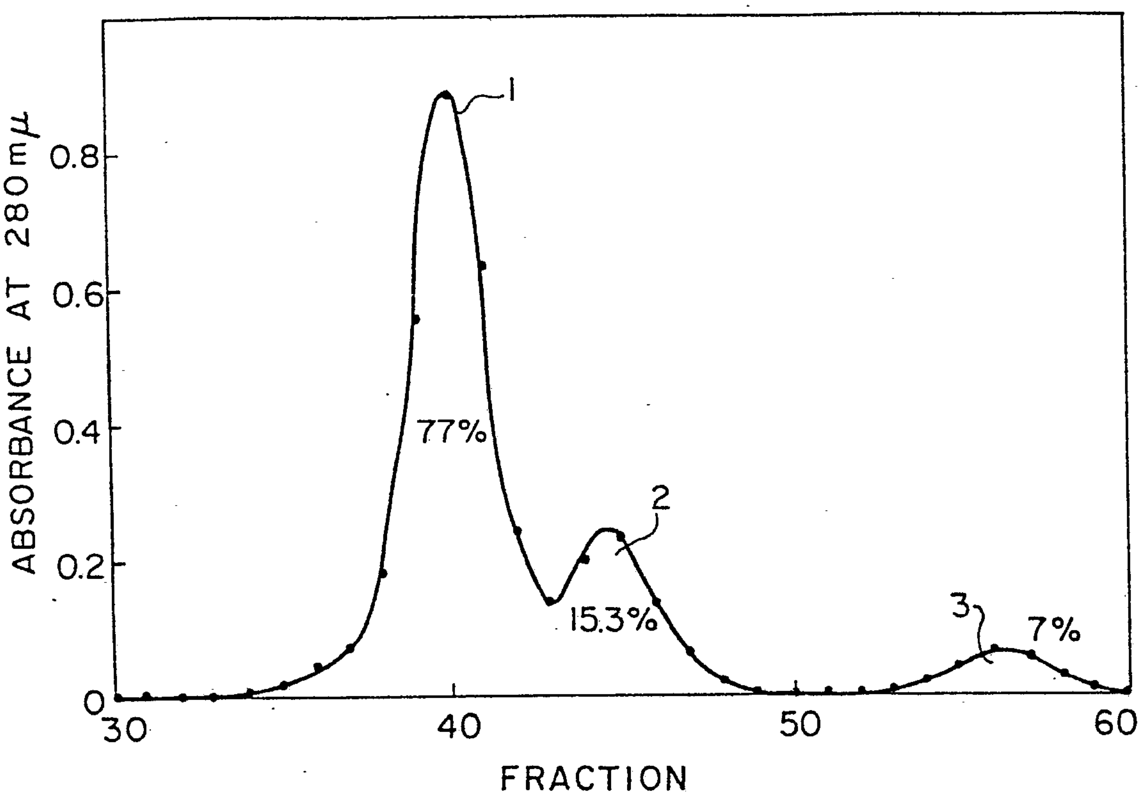
ABSORBANCE AT 280 mμ
0.8
0.6
0.4
0.2
0
30
40
50
60
FRACTION
1
2
3
77%
15.3%
7%

FIG. 6

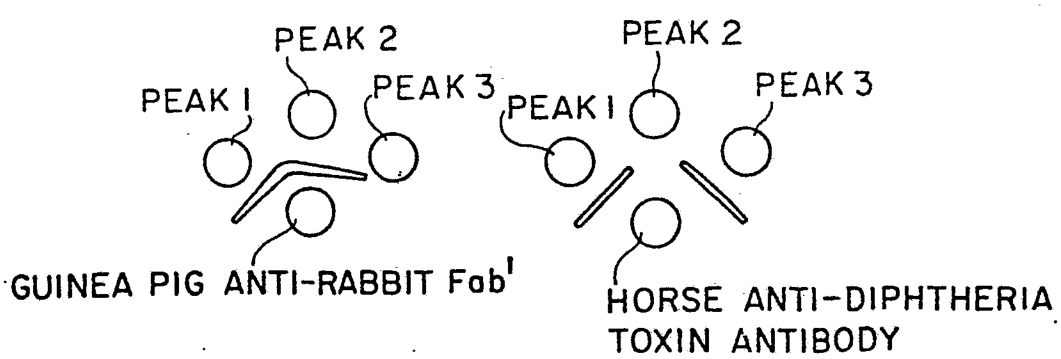
PEAK 1
PEAK 2
PEAK 3
GUINEA PIG ANTI-RABBIT Fab'
PEAK 1
PEAK 2
PEAK 3
HORSE ANTI-DIPHTHERIA TOXIN ANTIBODY

FIG. 7

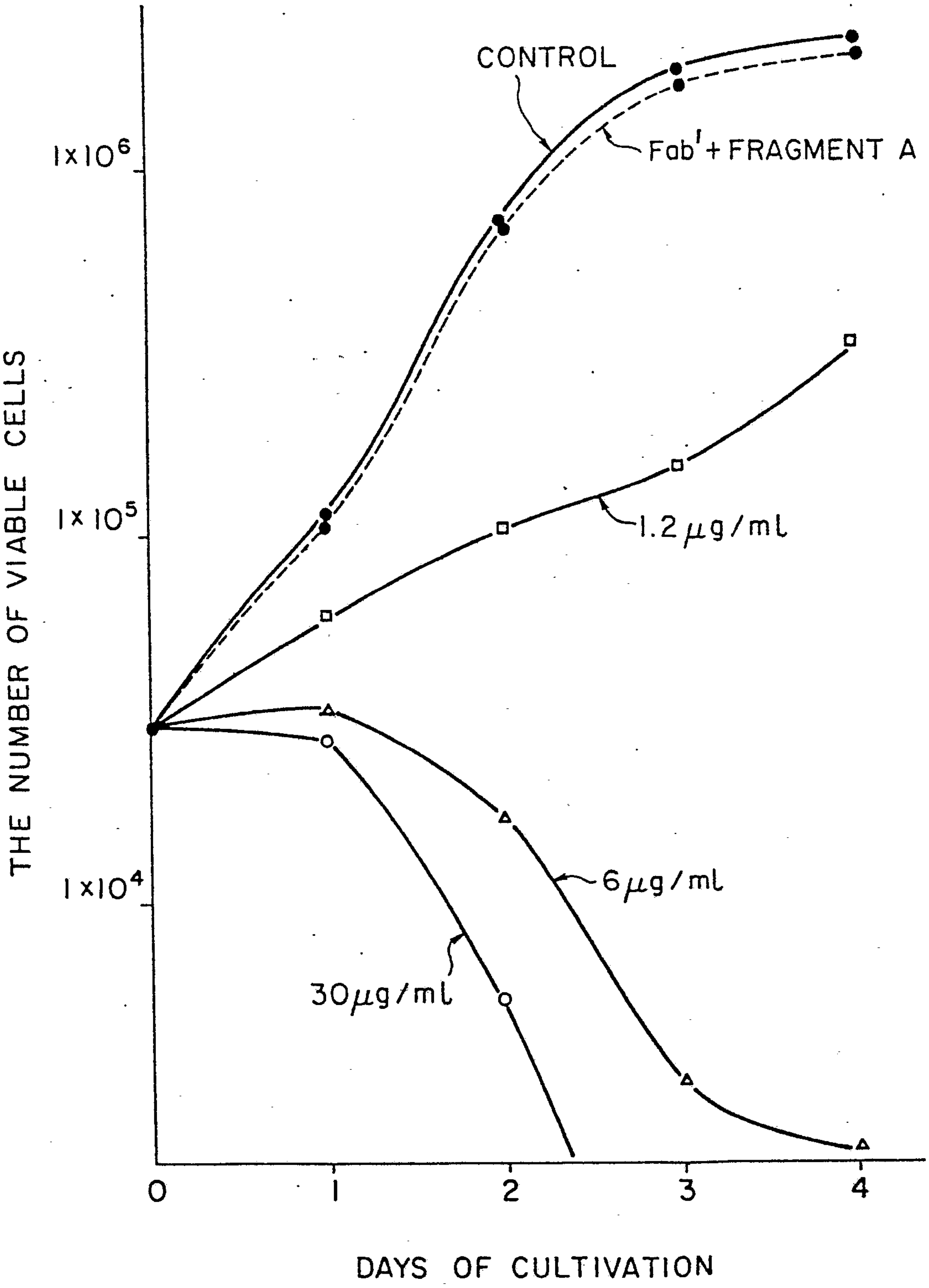
CONTROL
Fab' + FRAGMENT A
1.2 μg/ml
6 μg/ml
30 μg/ml
1x10⁶
1x10⁵
1x10⁴
THE NUMBER OF VIABLE CELLS
0
1
2
3
4
DAYS OF CULTIVATION